# EUROPÄISCHE PATENTANMELDUNG

(11) **EP 2 913 320 A1**
(43) Veröffentlichungstag der Anmeldung: **02.09.2015**
(21) Anmeldenummer: 14000707.1
(22) Anmeldetag: 27.02.2014
(51) Int. Cl.: C07C 41/01, C07C 43/04, B01J 8/02, F28D 7/02

(54) **Verfahren und Anlage zur Gewinnung von Oxygenaten mit kontinuierlichem Wärmetausch zwischen Einsatzstrom und Rohproduktstrom**

(71) Anmelder: Linde Aktiengesellschaft, 80331 München (DE)
(72) Erfinder: Fritz, Helmut, 81375 München (DE); Peschel, Andreas, 82515 Wolfratshausen (DE)
(74) Vertreter: Gellner, Bernd

(57) **Zusammenfassung**

Es wird ein Verfahren zur Gewinnung wenigstens eines Oxygenats vorgeschlagen, bei dem ein zumindest Wasserstoff und Kohlenmonoxid enthaltender Einsatzstrom (b, c) von einem ersten Temperaturniveau (T1) auf ein zweites Temperaturniveau (T2) erwärmt und in wenigstens einem Oxygenatreaktor (3) zu einem das wenigstens eine Oxygenat enthaltenden Rohproduktstrom (d, e) umgesetzt wird, der dem wenigstens einen Oxygenatreaktor (3) auf einem dritten Temperaturniveau (T3) entnommen wird, wobei der Einsatzstrom (b, c) zumindest teilweise durch Wärmetausch mit zumindest einem Teil des Rohproduktstroms (d, e) von dem ersten Temperaturniveau (T1) auf das zweite Temperaturniveau (T2) erwärmt wird und der Rohproduktstrom (d, e) dabei zumindest teilweise auf ein viertes Temperaturniveau (T4) abgekühlt wird. Für den Wärmetausch wird zumindest ein gewickelter Wärmetauscher (2) verwendet. Eine entsprechende Anlage (10) ist ebenfalls Gegenstand der Erfindung.

## Beschreibung

Die Erfindung betrifft ein Verfahren und eine Anlage zur Gewinnung wenigstens eines Oxygenats gemäß den Oberbegriffen der unabhängigen Patentansprüche.

### Stand der Technik

Unter Oxygenaten werden sauerstoffhaltige, von gesättigten Kohlenwasserstoffen abgeleitete Verbindungen verstanden, insbesondere Ether und Alkohole. Oxygenate kommen beispielsweise als Kraftstoffadditive zur Erhöhung der Oktanzahl und als Bleiersatz zum Einsatz (vgl. D. Barceló (Hrsg.): Fuel Oxygenates. In: D. Barceló und A.G. Kostianoy (Hrsg.): The Handbook of Environmental Chemistry, Bd. 5. Heidelberg: Springer, 2007). Die Zugabe von Oxygenaten in Kraftstoffen bewirkt unter anderem eine sauberere Verbrennung im Motor und verringert damit Emissionen.

Bei entsprechenden Oxygenaten handelt es sich typischerweise um Ether und Alkohole. Neben Methyl-tert-butylether (MTBE, engl. methyl tertiary butyl ether) kommen beispielsweise tert-Amylmethylether (TAME, tertiary amyl methyl ether), tert-Amylethylether (TAEE, tertiary amyl ethyl ether), Ethyl-tert-butylether (ETBE, ethyl tertiary butyl ether) und Diisopropylether (DIPE, diisopropyl ether) zum Einsatz. Als Alkohole werden beispielsweise Methanol, Ethanol und tert-Butanol (TBA, tertiary butyl alcohol) verwendet. Zu den Oxygenaten zählt insbesondere auch der nachfolgend erläuterte Dimethylether (DME, dimethyl ether). Die Erfindung ist nicht auf die Gewinnung von Kraftstoffadditiven beschränkt, sondern eignet sich in gleicher Weise zur Verwendung in Verfahren zur Herstellung anderer Oxygenate.

Bei Dimethylether handelt es sich um den strukturell einfachsten Ether. Dimethylether enthält zwei Methylgruppen als organische Reste. Dimethylether ist polar und findet herkömmlicherweise in flüssiger Form als Lösungsmittel Verwendung. Dimethylether kann ferner als Kühlmittel eingesetzt werden und beispielsweise herkömmliche Fluorchlorkohlenwasserstoffe ersetzen.

Neuerdings wird Dimethylether zunehmend als Ersatz für Brenngas (Flüssiggas) und herkömmliche Kraftstoffe wie Diesel eingesetzt. Aufgrund seiner vergleichsweise hohen Cetanzahl von 55 bis 60 müssen beispielsweise herkömmliche Dieselmotoren für den Betrieb mit Dimethylether nur geringfügig modifiziert werden. Dimethylether verbrennt vergleichsweise sauber und ohne Rußbildung. Wird Dimethylether aus Biomasse hergestellt, gilt er als sogenannter Biokraftstoff und kann daher steuerbegünstigt vermarktet werden.

Dimethylether kann entweder direkt aus Methanol oder indirekt aus Erd- oder Biogas erzeugt werden. Im letzteren Fall wird das Erd- oder Biogas zunächst zu Synthesegas reformiert. Synthesegas kann auch mittels anderer Verfahren, beispielsweise durch Pyrolyse von Abfällen oder Biomasse, gewonnen werden. Das Synthesegas wird klassischerweise zu Methanol und anschließend weiter zu Dimethylether umgesetzt. Die Gewinnung von Dimethylether aus Synthesegas ist thermodynamisch und wirtschaftlich vorteilhaft gegenüber einer Gewinnung aus Methanol.

Die vorliegende Erfindung erweist sich insbesondere als vorteilhaft bei der einstufigen oder direkten Gewinnung von Dimethylether aus Synthesegas. Unter einer einstufigen oder direkten Gewinnung wird hier eine Gewinnung ohne eine zwischengeschaltete Methanolabtrennung verstanden, wie sie in einer zweistufigen Gewinnung erfolgt. Die ablaufenden Reaktionen können jedoch auch in einer einstufigen Gewinnung Methanol als Zwischenprodukt bilden, das jedoch in dem oder den verwendeten Reaktoren zumindest zum Teil weiter zu Dimethylether reagiert. Entsprechende Verfahren sind seit längerem bekannt und werden auch unten näher erläutert.

Bei der einstufigen oder direkten Gewinnung von Dimethylether, jedoch auch bei der Gewinnung anderer Oxygenate aus Synthesegas, kommen exotherme katalytische Reaktionen, beispielsweise in Rohrreaktoren, zum Einsatz. Ein einem entsprechenden Reaktor entnommener Strom (nachfolgend als Rohproduktstrom bezeichnet) ist daher immer deutlich wärmer als ein in den Reaktor eingespeister Strom (nachfolgend als Einsatzstrom bezeichnet). Gleichzeitig muss der Einsatzstrom typischerweise auf Temperaturen oberhalb von 200 °C aufgeheizt werden, bevor er in den Reaktor eingespeist wird. Dies erfolgt zweckmäßigerweise zumindest teilweise gegen den Rohproduktstrom. Angestrebt wird in diesem Zusammenhang eine möglichst effektive Wärmeübertragung, die herkömmliche Verfahren und Anlagen jedoch nicht immer gewährleisten können.

Es besteht daher der Bedarf nach entsprechenden Verbesserungen bei Verfahren und Anlagen zur Gewinnung von Oxygenaten wie Dimethylether.

### Offenbarung der Erfindung

Vor diesem Hintergrund schlägt die vorliegende Erfindung ein Verfahren und eine Anlage zur Gewinnung wenigstens eines Oxygenats mit den Merkmalen der unabhängigen Patentansprüche vor. Bevorzugte Ausgestaltungen sind Gegenstand der abhängigen Patentansprüche sowie der nachfolgenden Beschreibung.

Vor der Erläuterung der Merkmale und Vorteile der vorliegenden Erfindung werden deren Grundlagen und die verwendeten Begriffe erläutert.

Ein Fluid (die Bezeichnung Fluid wird nachfolgend auch kurz für entsprechende Ströme, Fraktionen usw. verwendet) ist von einem anderen Fluid (das hier auch als Ausgangsfluid bezeichnet wird) abgeleitet oder aus einem solchen Fluid gebildet, wenn es zumindest einige in dem Ausgangsfluid enthaltene oder aus diesem erhaltene Komponenten aufweist. Ein in diesem Sinne abgeleitetes bzw. gebildetes Fluid kann aus dem Ausgangsfluid durch Abtrennen oder Abzweigen eines Anteils oder einer oder mehrerer Komponenten, Anreichern oder Abreichern bezüglich einer oder mehrerer Komponenten, chemisches oder physikalisches Umsetzen einer oder mehrerer Komponenten, Erwärmen, Abkühlen, Druckbeaufschlagen und dergleichen erhalten bzw. gebildet werden. Ein Strom kann auch beispielsweise einfach dadurch gebildet werden, dass er aus einem Speicherbehälter abgezogen wird.

Fluide können im hier verwendeten Sprachgebrauch reich oder arm an einer oder mehreren enthaltenen Komponenten sein, wobei reich für einen Gehalt von wenigstens 90%, 95%, 99%, 99,5%, 99,9%, 99,99% oder 99,999% und arm für einen Gehalt von höchstens 10%, 5%, 1%, 0,1%, 0,01% oder 0,001% auf molarer, Gewichts- oder Volumenbasis stehen kann. Sie können im hier verwendeten Sprachgebrauch angereichert oder abgereichert an einer oder mehreren Komponenten sein, wobei sich diese Begriffe auf einen entsprechenden Gehalt in einem Ausgangsfluid beziehen, aus dem das Fluid gebildet wurde. Das Fluid ist angereichert, wenn es zumindest den 1,1-fachen, 1,5-fachen, 2-fachen, 5-fachen, 10-fachen 100-fachen oder 1.000-fachen Gehalt, abgereichert, wenn es höchstens den 0,9-fachen, 0,5-fachen, 0,1-fachen, 0,01-fachen oder 0,001-fachen Gehalt einer entsprechenden Komponente, bezogen auf das Ausgangsfluid, enthält. Ein überwiegend eine oder mehrere Komponenten enthaltendes Fluid enthält diese eine oder mehreren Komponenten zu wenigstens 90%, 95%, 98% oder 99% bzw. ist reich an diesen.

Nachfolgend werden zur Charakterisierung von Drücken und Temperaturen die Begriffe Druckniveau und Temperaturniveau verwendet, wodurch zum Ausdruck gebracht werden soll, dass Drücke und Temperaturen nicht in Form exakter Druck- bzw. Temperaturwerte verwendet werden müssen, um ein erfinderisches Konzept zu verwirklichen. Jedoch bewegen sich derartige Drücke und Temperaturen typischerweise in bestimmten Bereichen, die beispielsweise ± 1%, 5%, 10%, 20% oder sogar 50% um einen Mittelwert liegen. Unterschiedliche Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste, beispielsweise aufgrund von Abkühlungseffekten, ein. Entsprechendes gilt für Temperaturniveaus. Bei den hier in bar angegebenen Druckniveaus handelt es sich um Absolutdrücke.

Ist nachfolgend kurz von einer Gewinnung von Dimethylether oder wenigstens eines Oxygenats die Rede, wird hierunter ein Verfahren verstanden, bei dem ein zumindest die bekannten Komponenten von Synthesegas enthaltender Einsatz, also ein Gasgemisch, das in geeigneten Anteilen zumindest Kohlenmonoxid und Wasserstoff enthält, zu einem Dimethylether enthaltenden Produktstrom umgesetzt wird. Ein entsprechender Produktstrom enthält aufgrund der nicht vollständigen Reaktion und aufgrund des Auftretens von Nebenreaktionen bei der Synthese von Dimethylether, insbesondere in Abhängigkeit von den Charakteristika der verwendeten Katalysatoren und den jeweiligen Gehalten der Komponenten des Synthesegases, nicht ausschließlich Dimethylether sondern weitere Verbindungen. Diese sind zumindest Methanol, Wasser, Kohlendioxid, Kohlenmonoxid und Wasserstoff, jedoch auch geringere Mengen an Methan, Ethan, organischen Säuren und höheren Alkoholen. Diese weiteren Verbindungen müssen zumindest teilweise abgetrennt werden, um einerseits nachfolgende Trennschritte zu ermöglichen und andererseits Dimethylether in der geforderten Reinheit zu gewinnen.

Das bei der Gewinnung von Dimethylether oder wenigstens eines Oxygenats verwendete Synthesegas kann auf beliebige Weise erzeugt werden, wie aus dem Stand der Technik bekannt (vgl. H. Hiller et al.: Gas Production. In: Ullmann's Encyclopedia of Industrial Chemistry, Onlineausgabe. Weinheim: Wiley-VCH, 2002. DOI: 10.1002/14356007.a12_169.pub2).

Die Erfindung kommt dabei bei Verfahren zur Anwendung, bei denen Einsätze wie Erdgas, Naphtha, Vakuumrückstände aus Raffinerien, Kohle, feste oder flüssige Abfälle und/oder Biomasse falls erforderlich vorbehandelt und/oder verdampft bzw. vergast, mit Prozessdampf vermischt und anschließend in wenigstens einem Reaktor (nachfolgend als Synthesegasreaktor bezeichnet) zumindest teilweise zu Synthesegas umgesetzt werden.

Zur Erzeugung von Synthesegas werden typischerweise ein- oder mehrstufige Verfahren eingesetzt. Hierbei kommen Rohrbündelreaktoren als Synthesegasreaktoren zum Einsatz, deren Reaktionsrohre herkömmlicherweise von außen befeuert und bei 850 bis 900 °C betrieben werden. Die Reaktionsrohre sind mit einem geeigneten Katalysator befüllt und werden von dem mit dem Prozessdampf vermischten Einsatz durchströmt. Die Erfindung kann neben der herkömmlichen Dampfreformierung (SMR, steam methane reforming) aber auch bei der autothermen Reformierung (ATR, autothermal reforming) und bei kombinierten Reformierungsverfahren aus herkömmlicher Dampfreformierung und authothermer Reformierung zum Einsatz kommen. Ein weiteres Einsatzgebiet der Erfindung sind Verfahren zur Dampfreformierung mit Kohlendioxidimport.

Dimethylether kann, wie eingangs bereits angesprochen, durch eine zweistufige Synthese aus Synthesegas über Methanol als Zwischenprodukt gewonnen werden. Entsprechende Verfahren sind beispielsweise ab Seite 171 im DME Handbook des Japan DME Forum, ISBN 978-4-9903839-0-9, 2007, beschrieben. Die zweistufige Gewinnung von Dimethylether aus Synthesegas zeichnet sich, wie erwähnt, dadurch aus, dass zunächst Methanol aus Synthesegas gewonnen wird, anschließend das nicht umgesetzte Synthesegas von den Kondensaten (Methanol und Wasser) abgetrennt wird, und das Methanol anschließend in einem weiteren Reaktor unter Gewinnung von Dimethylether und Wasser dehydratisiert wird.

In der Patentliteratur wird bereits 1973 (DE 23 62 944 A1, US 4 098 809 A) die direkte oder einstufige Gewinnung von Dimethylether aus Synthesegas beschrieben. Diese zeichnet sich durch eine gemeinsame Reaktionsstufe aus, in der aus Wasserstoff, Kohlenmonoxid und Kohlendioxid gemeinsam Methanol und Dimethylether gewonnen werden. Hierauf aufbauend wurden in der Literatur weitere Verfahren beschrieben.

In einem bekannten Kombinationsverfahren, dem sogenannten Topsoe-Prozess, wie er im DME Handbook ab Seite 185, insbesondere auf Seite 187 beschrieben ist, wird ein dualer Katalysator, mit dem sich sowohl Methanol als auch Dimethylether bilden lassen, eingesetzt. Es kommen mindestens zwei Reaktoren ohne zwischengeschaltete Auftrennung zum Einsatz, wobei ein erster Reaktor isotherm gekühlt und ein zweiter Reaktor adiabat betrieben wird. Es erfolgt eine parallele Produktion von Dimethylether und Methanol, wobei das Methanol in einem weiteren Reaktor nach Auftrennung der Komponenten zu Dimethylether umgesetzt werden kann.

Die Direktsynthese von Dimethylether kann auch beispielsweise im Slurrybetrieb und bei vergleichsweise geringen Stöchiometriezahlen erfolgen. Hierdurch kommt es zur Bildung von Kohlendioxid als Nebenprodukt, das herkömmlicherweise von den jeweils nicht umgesetzten Verbindungen abgetrennt werden muss, um letztere als Recycle der Reaktion erneut zuführen zu können. Die genannten Reaktionen laufen bei vergleichsweise geringen Stöchiometriezahlen nur bei niedrigem Kohlendioxidgehalt mit befriedigender Ausbeute ab.

Die Erfindung kann auch mit besonderem Vorteil bei von der Anmelderin entwickelten Verfahren und Vorrichtungen zur Gewinnung von Dimethylether zum Einsatz kommen, bei denen ein Einsatzstrom mit Wasserstoff, Kohlenmonoxid und Kohlendioxid entsprechend einer Stöchiometriezahl von mehr als zwei und einem vergleichsweise hohen Anteil an Kohlendioxid verwendet wird. Der oder die hierbei eingesetzten Reaktoren werden unter ausschließlich isothermen Bedingungen betrieben.

Den Verfahren zur Gewinnung von Dimethylether und anderer Oxygenate ist, wie erwähnt, gemeinsam, dass diese mehr oder weniger stark exotherm ablaufen. Da die jeweils verwendeten Reaktionen vorteilhafterweise zumindest teilweise isotherm geführt werden sollten, ist eine entsprechende Wärmeabfuhr, beispielsweise unter Verwendung von Kühlwasser, erforderlich.

Zur Gewinnung von Dimethylether und anderer Oxygenate werden typischerweise ebenfalls Rohrbündelreaktoren eingesetzt, deren mit Katalysator befüllte und von einem zumindest die Komponenten des eingesetzten Synthesegases enthaltenden Einsatzstrom durchströmte Reaktionsrohre von außen entsprechend gekühlt werden. Neben Rohrbündelreaktoren können grundsätzlich auch andere Reaktortypen, beispielsweise Plattenreaktoren, zum Einsatz kommen.

In jedem Fall wird ein zumindest die Komponenten von Synthesegas enthaltender Einsatzstrom durch einen Reaktionsraum wenigstens eines Reaktors (nachfolgend als Oxygenatreaktor bezeichnet) geführt, der baulich von einem Kühlraum des wenigstens einen Oxygenatreaktors getrennt ist. Der Reaktionsraum kann dabei in eine Vielzahl von Teilräumen unterteilt sein, beispielsweise die mit dem verwendeten Katalysator verwendeten Reaktionsrohre eines Rohrbündelreaktors und/oder in die plattenförmigen Kammern eines Plattenreaktors. Entsprechendes gilt auch für den Kühlraum.

Der Kühlraum wird typischerweise von einem insbesondere wässrigen Kühlfluid durchströmt. Herkömmlicherweise handelt es sich hierbei um Kühlwasser, das in einem Kühlwasserkreislauf geführt wird. Der im Synthesegasreaktor eingesetzte Dampf wird hingegen als Prozessdampf in einem von dem Kühlwasserkreislauf getrennten, separaten Dampferzeugungssystem bereitgestellt.

### Vorteile der Erfindung

Die vorliegende Erfindung geht von einem Verfahren zur Gewinnung wenigstens eines Oxygenats, beispielsweise Dimethylether, aus, bei dem ein zumindest Wasserstoff und Kohlenmonoxid enthaltender Einsatzstrom, beispielsweise ein druckbeaufschlagter Synthesegasstrom, von einem ersten Temperaturniveau auf ein zweites Temperaturniveau erwärmt und in wenigstens einem Oxygenatreaktor zu einem das wenigstens eine Oxygenat enthaltenden Rohproduktstrom umgesetzt wird. Verfahrensvarianten umfassen hierbei die Verwendung mehrerer Einsatzströme, mehrerer Oxygenatreaktoren und/oder die Gewinnung mehrerer Rohproduktströme, die jeweils gleich oder unterschiedlich behandelt werden können.

Unter einem Rohprodukt wird hier der Abstrom eines entsprechenden Oxygenatreaktors verstanden, der anschließend beispielsweise bekannten Aufreinigungsstufen, beispielsweise zur Gewinnung von reinem Dimethylether, zugeführt wird. Der Rohproduktstrom wird dem wenigstens einen Oxygenatreaktor auf einem dritten Temperaturniveau entnommen. Wie insoweit bekannt, wird im Rahmen des erfindungsgemäßen Verfahrens der Einsatzstrom zumindest teilweise durch Wärmetausch mit zumindest einem Teil des Rohproduktstroms von dem ersten Temperaturniveau auf das zweite Temperaturniveau erwärmt. Dies bewirkt umgekehrt, dass der Rohproduktstrom dabei zumindest teilweise auf ein viertes Temperaturniveau abgekühlt wird. Wie eingangs erläutert, umfassen bekannte Verfahren zur Gewinnung von Oxygenaten typischerweise die Verwendung von exothermen, katalytischen Reaktionen, so dass sich die überschüssige Wärme eines entsprechenden Rohproduktstroms für die Erwärmung des Einsatzstroms einsetzen lässt.

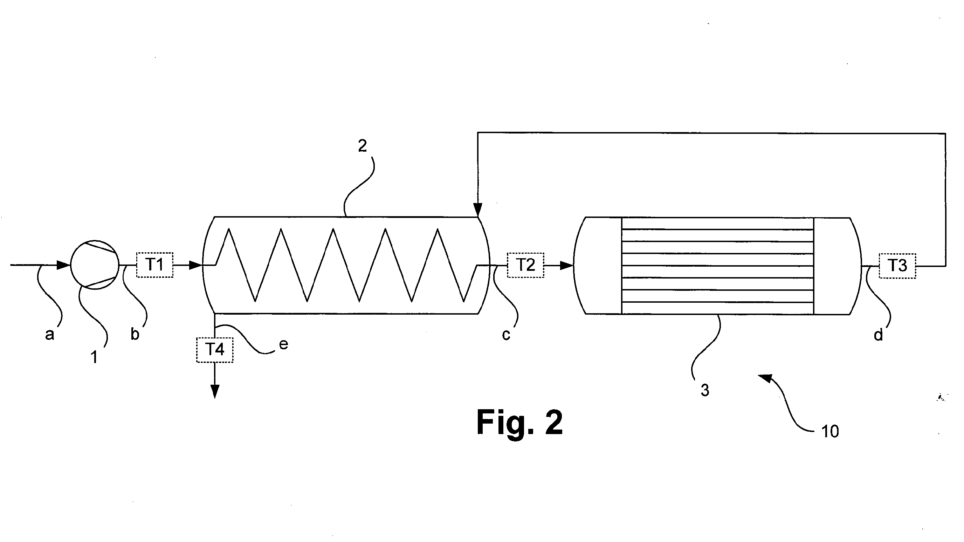

Wie auch unter Bezugnahme auf die Figur 2 erläutert, stoßen herkömmliche Verfahren hierbei an ihre Grenzen. Aus energetischer Sicht ist es wünschenswert, möglichst die gesamte Wärme aus dem Rohproduktstrom auf den Einsatzstrom zu übertragen, so dass sich die Temperaturen hierbei möglichst weitgehend angleichen. Dies betrifft im Rahmen der vorliegenden Erfindung das erste und das vierte Temperaturniveau bzw. das zweite und das dritte Temperaturniveau. Ein idealer Wärmeaustausch würde also bewirken, dass einander die erste und vierte bzw. die zweite und dritte Temperatur entsprechen. Dies ist technisch nicht möglich. Insbesondere bei der Verwendung herkömmlicher Plattenwärmetauscher kann jeweils nur ein stufenweiser Wärmetausch in einem begrenzten Temperaturumfang realisiert werden. Dies kann in einem Wärmeaustauschdiagramm in Form sogenannter Treppenzüge veranschaulicht werden. Wäre im Rahmen eines der Erfindung zugrunde liegenden Verfahrens beispielsweise eine Annäherung zwischen dem ersten und vierten Temperaturniveau bzw. dem zweiten und dem dritten Temperaturniveau auf einen Wert von weniger als 30 °C Unterschied erwünscht, wäre eine Vielzahl von einzelnen Plattenwärmetauschern erforderlich, die seriell betrieben werden und jeweils nur eine geringe Wärmemenge austauschen. Das Verfahren wäre damit nicht wirtschaftlich. Hier setzt die vorliegende Erfindung an und schlägt vor, für den Wärmetausch zumindest einen gewickelten Wärmetauscher zu verwenden.

Gewickelte Wärmetauscher (auch als spiralgewickelte Wärmetauscher bezeichnet, engl. coil wound heat exchangers) sind grundsätzlich bekannt. Sie werden jedoch herkömmlicherweise zum Wärmetausch bei kryogenen Temperaturen, typischerweise bei Temperaturen deutlich unterhalb von 0 °C, eingesetzt. Merkmale von gewickelten Wärmetauschern sind beispielsweise bei K. Thulukkanam: Heat Exchanger Design Handbook. 2. Aufl. Boca Raton: CRC Press, 2013, beschrieben.

Gewickelte Wärmetauscher sind vergleichsweise kompakt und zuverlässig. Sie ermöglichen breite Temperatur- und Druckbereiche denen insbesondere herkömmliche Plattenwärmetauscher und/oder geschraubte Wärmetauscher nicht standhalten können. Gewickelte Wärmetauscher eigenen sich sowohl für Ein- als auch Zweiphasenströme. In gewickelten Wärmetauschern können auch mehrere Ströme gegeneinander geführt werden. Durch die Verwendung gewickelter Rohrschlangen werden insbesondere Spannungen bei großen Temperaturunterschieden, insbesondere bei der Inbetriebnahme oder Außerbetriebnahme einer entsprechenden Anlage verringert. Im Rahmen der vorliegenden Erfindung ermöglicht die Verwendung eines gewickelten Wärmetauschers die Realisierung eines nahezu vollständigen Wärmeaustauschs zwischen den beteiligten Strömen, so dass beinahe die gesamte bei der exothermen Reaktion in dem wenigstens einen Oxygenatreaktor frei werdende Wärme auf den Einsatzstrom übertragen werden kann. Trotz der höheren Investitionskosten für gewickelte Wärmetauscher ermöglicht das erfindungsgemäße Verfahren daher Kosteneinsparungen aufgrund des energetisch günstigeren Betriebs. Gewickelte Wärmetauscher erweisen sich, wie erwähnt, ferner als weniger wartungsanfällig als die herkömmlicherweise verwendeten Plattenwärmetauscher.

Im Rahmen der vorliegenden Erfindung liegt das vierte Temperaturniveau insbesondere 5 bis 50 °C oberhalb des ersten Temperaturniveaus und das dritte Temperaturniveau 5 bis 50 °C oberhalb des zweiten Temperaturniveaus. Insbesondere betragen hierbei die Unterschiede zwischen dem vierten und dem ersten Temperaturniveau bzw. dem dritten und dem zweiten Temperaturniveau weniger als 30 °C. Wie erwähnt, ist also ein nahezu vollständiger Wärmeaustausch möglich. Derartige geringe Temperaturunterschiede können mit Plattenwärmetauschern nicht oder nur mit beträchtlichem Aufwand, d.h. einer großen Anzahl an Wärmetauschern erreicht werden.

Im Rahmen der vorliegenden Erfindung beträgt das erste Temperaturniveau und das vierte Temperaturniveau jeweils vorteilhafterweise 0 bis 100 °C und das zweite Temperaturniveau und das dritte Temperaturniveau betragen jeweils 200 bis 300 °C. Bei Verfahren zur Gewinnung von Dimethylether wird dabei beispielsweise ein Einsatzstrom von einem ersten Temperaturniveau bei 30 °C auf ein zweites Temperaturniveau bei 250 °C erwärmt bzw. ein Rohproduktstrom von einem dritten Temperaturniveau bei 280 °C auf ein viertes Temperaturniveau bei 60 °C abgekühlt. Insbesondere beträgt das erste Temperaturniveau damit 20 bis 40 °C und das vierte Temperaturniveau 50 bis 70 °C. Entsprechend beträgt das zweite Temperaturniveau vorteilhafterweise 240 bis 260 °C. Das dritte Temperaturniveau beträgt vorteilhafterweise 270 bis 290 °C.

Im Rahmen der vorliegenden Erfindung wird der Einsatzstrom vorteilhafterweise auf einem Druckniveau von 20 bis 100 bar in den wenigstens einen Oxygenatreaktor eingespeist. Bei der Herstellung von Dimethylether kommen dabei beispielsweise Drücke von 30 bis 70 bar zum Einsatz.

Das erfindungsgemäße Verfahren schließt die Verwendung weiterer Wärmetauscher zur Erwärmung des Einsatzstroms nicht aus. Dabei kann der Einsatzstrom unter Verwendung wenigstens eines weiteren Wärmetauschers entweder auf das erste Temperaturniveau und/oder von dem zweiten Temperaturniveau auf ein weiteres Temperaturniveau erwärmt werden. Ein Wärmetauscher bzw. Erhitzer, der zur Erwärmung von dem zweiten Temperaturniveau auf ein weiteres Temperaturniveau verwendet wird, wird auch als sogenannter Spitzenerhitzer bezeichnet.

Vorteilhafterweise wird der Einsatzstrom im Rahmen des erfindungsgemäßen Verfahrens unter Verwendung wenigstens eines Synthesegasreaktors zur Durchführung eines Dampfreformierungsverfahrens, eines autothermen Reformierungsverfahrens, eines kombinierten Verfahrens aus einem Dampfreformierungsverfahren und einem autothermen Reformierungsverfahren und/oder eines Dampfreformierungsverfahrens mit Kohlendioxidimport bereitgestellt. Im Rahmen der vorliegenden Erfindung eignen sich damit sämtliche Verfahren zur Bereitstellung von Synthesegas, die aus dem Stand der Technik bekannt sind.

Die Erfindung eignet sich zur Gewinnung von Dimethylether, jedoch auch zur Gewinnung anderer Oxygenate wie beispielsweise Methyl-tert-butylether, tert-Amylmethylether, tert-Amylethylether, Ethyl-tert-butylether, Diisopropylether, Methanol, Ethanol und/oder tert-Butanol.

Eine erfindungsgemäß ebenfalls vorgesehene Anlage zur Gewinnung wenigstens eines Oxygenats umfasst Mittel, die dafür eingerichtet sind, einen zumindest Wasserstoff und Kohlenmonoxid enthaltenden Einsatzstrom von einem ersten Temperaturniveau auf ein zweites Temperaturniveau zu erwärmen, wenigstens einen Oxygenatreaktor, der dafür eingerichtet ist, den erwärmten Einsatzstrom zu einem das wenigstens eine Oxygenat enthaltenden Rohproduktstrom umzusetzen, und Mittel, die dafür eingerichtet sind, den Rohproduktstrom dem wenigstens einen Oxygenatreaktor auf einem dritten Temperaturniveau zu entnehmen. Ferner sind Mittel vorgesehen, die dafür eingerichtet sind, den Einsatzstrom zumindest durch Wärmetausch mit zumindest einem Teil des Rohproduktstroms von dem ersten Temperaturniveau auf das zweite Temperaturniveau zu erwärmen und den Rohproduktstrom dabei zumindest teilweise auf ein viertes Temperaturniveau abzukühlen. Erfindungsgemäß weist eine derartige Anlage zumindest einen gewickelten Wärmetauscher auf, der für den erwähnten Wärmetausch eingerichtet ist.

Vorteilhafterweise umfasst eine derartige Anlage sämtliche Mittel, die sie zur Durchführung eines Verfahrens befähigen, wie es zuvor erläutert wurde. Auf die genannten Vorteile wird daher ausdrücklich verwiesen.

Grundlagen und Ausführungsformen der Erfindung werden unter Bezugnahme auf die beigefügten Zeichnungen näher erläutert.

Kurze Beschreibung der Zeichnungen
Figur 1 zeigt einen Wärmeaustausch in Form eines Wärmeaustauschdiagramms.
Figur 2 veranschaulicht eine Anlage gemäß einer Ausführungsform der Erfindung.

### Ausführungsformen der Erfindung

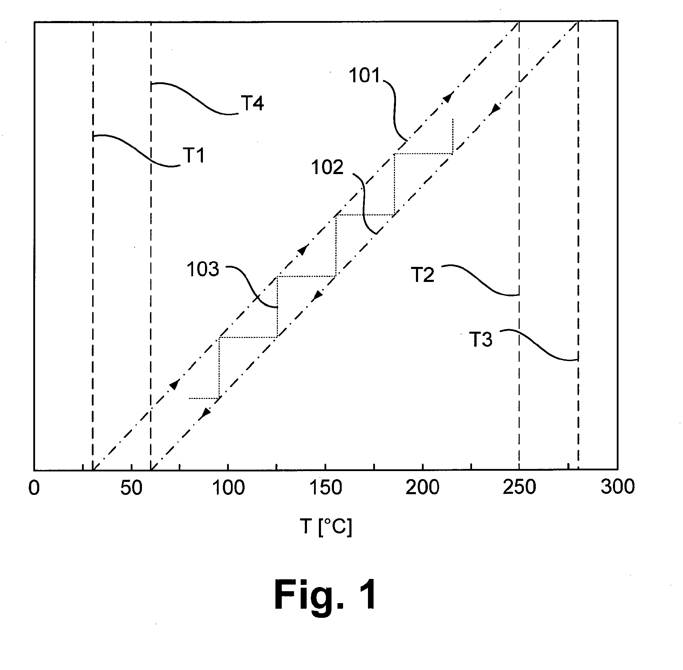

In Figur 1 ist ein Wärmeaustausch zwischen zwei Strömen in Form eines QT- bzw. Wärmeaustauschdiagramms veranschaulicht, in welchem eine Wärmemenge Q (ohne Einheiten) auf der Ordinate gegenüber einer Temperatur T in °C auf der Abszisse aufgetragen ist. Mit den Graphen 101 und 102 ist veranschaulicht, wie ein erster Strom (Graph 101) von einem ersten Temperaturniveau T1 von beispielsweise 30 °C auf ein zweites Temperaturniveau T2 von beispielsweise 250 °C erwärmt und ein zweiter Strom (Graph 102) von einem dritten Temperaturniveau T3 von beispielsweise 280 °C auf ein viertes Temperaturniveau T4 von beispielsweise 60 °C abgekühlt wird. Die jeweilige Wärmeaufnahme bzw. Abgabe ist durch Pfeile veranschaulicht.

Wie eingangs erläutert, ist mit herkömmlicherweise verwendeten Plattenwärmetauschern dabei jeweils nur ein stufenweiser Wärmeaustausch möglich, weshalb die Graphen 101 und 102 einander nicht beliebig angenähert werden können. Dies ist mit Linie 103 veranschaulicht, die üblicherweise auch als "Treppenzug" bezeichnet wird.

Aus energetischen Gründen ist jedoch eine möglichst geringe Distanz zwischen den Graphen 101 und 102 und damit zwischen den Temperaturen T1 und T4 einerseits und T2 und T3 andererseits erwünscht. Mit anderen Worten soll jeweils ein möglichst vollständiger Wärmeaustausch zwischen den beteiligten Strömen erfolgen. Bei der Verwendung von Plattenwärmetauschern ist dies jedoch nicht möglich bzw. wirtschaftlich nicht sinnvoll, da hierzu eine sehr große Anzahl Wärmetauscher mit entsprechend geringen Temperaturunterschieden erforderlich wäre. Es müsste also, mit anderen Worten, eine Vielzahl von Wärmetauschern hintereinander geschaltet werden, die jeweils nur eine geringfügige Erwärmung bzw. Abkühlung der beteiligten Ströme bewirken würden.

Im Rahmen der vorliegenden Erfindung wird dieses Problem durch die mehrfach erwähnte Verwendung eines gewickelten Wärmetauschers gelöst. Wie erwähnt, ermöglicht ein gewickelter Wärmetauscher einen kontinuierlichen Wärmetausch zwischen Strömen, wobei ein Temperaturunterschied - im vorliegenden Beispiel zwischen den Temperaturen T1 und T4 einerseits und T2 und T3 andererseits - von beispielsweise weniger als 30 °C ermöglicht wird.

Figur 2 veranschaulicht eine Anlage gemäß einer Ausführungsform der Erfindung in Form eines Prozessflussdiagramms. Die Anlage ist insgesamt mit 10 bezeichnet.

Die Anlage 10 wird im dargestellten Beispiel mit einem Einsatzstrom a beschickt, der zumindest Wasserstoff und Kohlenmonoxid, also die üblichen Komponenten von Synthesegas, enthält. Der Einsatzstrom a kann auch weitere Komponenten, insbesondere Kohlendioxid, enthalten. Der Einsatzstrom a wird in einem Verdichter 1 druckerhöht, beispielsweise auf einen Druck von 20 bis 100 bar, beispielsweise auf 40 bis 70 bar. Der entsprechende verdichtete Einsatzstrom, nun mit b bezeichnet, liegt nach der Verdichtung in dem Verdichter 1 auf einem ersten Temperaturniveau, das hier mit T1 bezeichnet ist, vor. Das erste Temperaturniveau T1 liegt bei oder, aufgrund der zugeführten Verdichtungswärme, oberhalb der Umgebungstemperatur.

Der Strom b wird auf dem ersten Temperaturniveau T1 in der dargestellten Ausführungsform der Erfindung einem gewickelten Wärmetauscher 2 zugeführt und durchläuft in diesem einen oder mehrere spiralförmig gewickelte Wärmetauscherkanäle. Der Strom b wird in dem gewickelten Wärmetauscher 2 mit einem Strom d (siehe unten) erwärmt. Stromab des gewickelten Wärmetauschers 2 liegt der Strom b, der nun mit c bezeichnet ist, auf einem zweiten Temperaturniveau T2 vor. Eine weitere Erwärmung stromauf und/oder stromab des gewickelten Wärmetauschers 2 ist möglich, beispielsweise in einem sogenannten Spitzenerhitzer stromab des gewickelten Wärmetauschers 2. Der Strom c wird in einen Oxygenatreaktor 3, beispielsweise einen Rohrbündelreaktor 3, eingespeist. Anstelle des in Figur 2 dargestellten einzelnen Rohrbündelreaktors 3 können insbesondere auch mehrere Reaktoren vorgesehen sein, die parallel oder seriell betrieben werden können. Der oder die Rohrbündelreaktoren 3 und/oder andere einsetzbare Reaktortypen können isotherm gekühlt und/oder adiabatisch betrieben werden.

Stromab des dargestellten Oxygenatreaktors 3 liegt der Strom c auf einem nochmals höheren, dritten Temperaturniveau T3 vor, da die Reaktionen in dem oder den Rohrbündelreaktoren 3 exotherm ablaufen. Der nun mit d bezeichnete Strom eignet sich daher in besonderer Weise zur Erwärmung des Stroms b und wird daher als Heizmedium in dem gewickelten Wärmetauscher 2 verwendet. Stromab des gewickelten Wärmetauschers 2 liegt der Strom d, nun mit e bezeichnet, auf einem reduzierten Temperaturniveau T4 vor, das jedoch systembedingt oberhalb des ersten Temperaturniveaus T1 liegt. In entsprechender Weise liegt auch das Temperaturniveau T2 systembedingt oberhalb des zweiten Temperaturniveaus T2, auf welchem der Strom c den gewickelten Wärmetauscher verlässt.

## Patentansprüche

1. Verfahren zur Gewinnung wenigstens eines Oxygenats, bei dem ein zumindest Wasserstoff und Kohlenmonoxid enthaltender Einsatzstrom (b, c) von einem ersten Temperaturniveau (T1) auf ein zweites Temperaturniveau (T2) erwärmt und in wenigstens einem Oxygenatreaktor (3) zu einem das wenigstens eine Oxygenat enthaltenden Rohproduktstrom (d, e) umgesetzt wird, der dem wenigstens einen Oxygenatreaktor (3) auf einem dritten Temperaturniveau (T3) entnommen wird, wobei der Einsatzstrom (b, c) zumindest teilweise durch Wärmetausch mit zumindest einem Teil des Rohproduktstroms (d, e) von dem ersten Temperaturniveau (T1) auf das zweite Temperaturniveau (T2) erwärmt wird und der Rohproduktstrom (d, e) dabei zumindest teilweise auf ein viertes Temperaturniveau (T4) abgekühlt wird, **dadurch gekennzeichnet, dass** für den Wärmetausch zumindest ein gewickelter Wärmetauscher (2) verwendet wird.

2. Verfahren nach Anspruch 1, bei dem das vierte Temperaturniveau (T4) 5 bis 50 °C oberhalb des ersten Temperaturniveaus (T1) und das dritte Temperaturniveau (T3) 5 bis 50 °C oberhalb des zweiten Temperaturniveaus (T2) liegt.

3. Verfahren nach Anspruch 2, bei dem das erste Temperaturniveau (T1) und das vierte Temperaturniveau (T4) 0 bis 100 °C und das zweite Temperaturniveau (T2) und das dritte Temperaturniveau (T3) 200 bis 300 °C betragen.

4. Verfahren nach einem der vorstehenden Ansprüche, bei dem der Einsatzstrom (b, c) auf einem Druckniveau von 20 bis 100 bar in den wenigstens einen Oxygenatreaktor (3) eingespeist wird.

5. Verfahren nach einem der vorstehenden Ansprüche, bei dem der Einsatzstrom (b, c) unter Verwendung wenigstens eines weiteren Wärmetauschers auf das erste Temperaturniveau (T1) und/oder von dem zweiten Temperaturniveau (T2) auf ein weiteres Temperaturniveau erwärmt wird.

6. Verfahren nach einem der vorstehenden Ansprüche, bei dem der Einsatzstrom (b, c) unter Verwendung wenigstens eines Synthesegasreaktors (2) zur Durchführung eines Dampfreformierungsverfahrens, eines autothermen
Reformierungsverfahrens, eines kombinierten Verfahrens aus einem Dampfreformierungsverfahren und einem autothermen Reformierungsverfahren und/oder eines Dampfreformierungsverfahrens mit Kohlendioxidimport bereitgestellt wird.

7. Verfahren nach einem der vorstehenden Ansprüche, bei dem Dimethylether, Methyl-tert-butylether, tert-Amylmethylether, tert-Amylethylether, Ethyl-tert-butylether, Diisopropylether, Methanol, Ethanol und/oder tert-Butanol als das wenigstens eine Oxygenat gewonnen wird.

8. Anlage (10) zur Gewinnung wenigstens eines Oxygenats, mit Mitteln, die dafür eingerichtet sind, einen zumindest Wasserstoff und Kohlenmonoxid enthaltenden Einsatzstrom (b, c) von einem ersten Temperaturniveau (T1) auf ein zweites Temperaturniveau (T2) zu erwärmen, wenigstens einem Oxygenatreaktor (3), der dafür eingerichtet ist, den erwärmten Einsatzstrom (b, c) zu einem das wenigstens eine Oxygenat enthaltenden Rohproduktstrom (d, e) umzusetzen, und Mitteln, die dafür eingerichtet sind, den Rohproduktstrom (d, e) dem wenigstens einen Oxygenatreaktor (3) auf einem dritten Temperaturniveau (T3) zu entnehmen, wobei Mittel vorgesehen sind, die dafür eingerichtet sind, den Einsatzstrom (b, c) zumindest teilweise durch Wärmetausch mit zumindest einem Teil des Rohproduktstroms (d, e) von dem ersten Temperaturniveau (T1) auf das zweite Temperaturniveau (T2) zu erwärmen und den Rohproduktstrom (d, e) dabei zumindest teilweise auf ein viertes Temperaturniveau (T4) abzukühlen, **dadurch gekennzeichnet, dass** für den Wärmetausch zumindest ein gewickelter Wärmetauscher (2) bereitgestellt ist.

9. Anlage (100) nach Anspruch 8, die zur Durchführung eines Verfahrens nach einem der Ansprüche 1 bis 7 eingerichtet ist.
